# EUROPEAN PATENT APPLICATION

(11) **EP 3 492 074 A1**
(43) Date of publication of application: **05.06.2019**
(21) Application number: 17837202.5
(22) Date of filing: 31.07.2017
(51) Int. Cl.: A61K 9/70, A61K 9/00, A61M 37/00, A61L 31/14, A61L 31/04, A61L 31/16

(54) **MICRONEEDLE PATCH AND MANUFACTURING METHOD THEREFOR**

(30) Priority: 01.08.2016 KR 20160098124
(71) Applicant: Pusan National University Industry - University Cooperation Foundation, Busan 46241 (KR)
(72) Inventor: YANG, Seung Yun, Miryang-si Gyeongsangnam-do 50441 (KR); SEONG, Keum Yong, Busan 48790 (KR)
(74) Representative: Gulde & Partner
(86) International application number: PCT/KR2017/008237
(87) International publication number: WO 2018/026151

(57) **Abstract**

In a microneedle patch and a manufacturing method therefor of the present invention, the microneedle patch of the present invention comprises: a patch layer which is a flat surface; and a microneedle disposed on one surface of the patch layer, comprising an air-pocket therein, and formed of a polymer having at least one of swellability and solubility.

## Description

### [Technical Field]

The present invention relates to a microneedle patch, and more particularly, to a microneedle patch and a manufacturing method therefor.

### [Background Art]

A microneedle means a material having a micrometer-sized needle shape, and may directly deliver drugs into the skin through the skin and thus has a high interest as a drug delivery material. The microneedle may be manufactured using various materials such as metal, glass, silicone, polymer, etc., and recently, researches on a microneedle using a biodegradable polymer have been actively conducted.

The microneedle formed of the biodegradable polymer is a microneedle which is biodegraded after the whole or a part (tip) of the microneedle is inserted into the skin to deliver the drugs into the body. However, the microneedle manufactured according to a conventional method has a problem that upon insertion of the skin, the skin tissue damage caused by inserting the microneedle into the skin is large, and particularly, there is a high risk of infection through a hole on the skin surface formed by the microneedle. In addition, there is a problem that a polymer microneedle inserted into the skin is rapidly dissolved or decomposed in the body, and thus continuous drug release is difficult.

Therefore, it is required to develop an insertion type microneedle which can be easily manufactured, has a low risk of infection after insertion into the skin, and enables continuous drug release in order to improve the problems of the conventional implanted microneedles.

### [Disclosure]

### [Technical Problem]

An object of the present invention is to provide a microneedle patch capable of preventing tissue damage and infection and delivering drugs.

Another object of the present invention is to provide a manufacturing method for the microneedle patch.

### [Technical Solution]

An aspect of the present invention provides a microneedle patch including a patch layer which is a flat layer; and a microneedle which is disposed on one surface of the patch layer, includes an air-pocket therein, and formed of a polymer having at least one of swellability and solubility.

In one embodiment, the air-pocket may be disposed in a lower end of the microneedle which is in contact with the patch layer.

In one embodiment, the patch layer may include a needle layer on one surface of the patch layer, and the microneedle may be disposed on an opposite surface to one surface of the needle layer which is in contact with the patch layer. In one embodiment, the air-pocket may be disposed in a lower end of the microneedle which is in contact with the needle layer. At this time, in the lower end of the microneedle, the air-pocket may be formed in the needle layer which is in contact with the lower end of the microneedle and the patch layer may be exposed to the air-pocket. At this time, in the lower end of the microneedle, a height of the air-pocket protruding from the exposed patch layer may be equal to or larger than a thickness of the skin horny layer.

At this time, the protruding height of the air-pocket may be 10 µm to 300 µm.

In one embodiment, after the insertion into the skin, the microneedle inserted into the skin may be constituted so that the side wall of the microneedle where the air-pocket is located is cut by a force of fixing the microneedle to the skin by swelling and a force of removing the microneedle patch from the skin.

In one embodiment, the polymer may be at least one material selected from the group consisting of hyaluronic acid, alginic acid, pectin, carrageenan, chondroitin (sulfate), dextran (sulfate), chitosan, polylysine, collagen, gelatin, carboxymethyl chitin, fibrin, agarose, pullulan, cellulose, polyethylene oxide, poly(N-)isopropylacrylamide (PNIPAAm), polyacrylamide (PAAm), polymethacrylic acid, polymaleic acid, polyvinyl alcohol (PVA), polyethylene oxide (PEO), poly(N-)vinyl pyrrolidone (PVP), poly(methyl methacrylate-co-hydroxylethyl methacrylate) (Poly(MMA-co-HEMA)), poly(acrylonitrile-arylsulfonate), poly(glucosyloxyethyl methacrylate-sulfate) (P(GEMA)), polyethylene oxide (PEO)-polyester derivatives, a polyethylene oxide-polypropylene oxide-polyethylene oxide (PEO-PPO-PEO) ternary block copolymer, N-carboxyanhydride, polystyrene, and any copolymer of monomers forming these polymers.

In one embodiment, the number average molecular weight of the polymer is 10,000 or more.

In one embodiment, after the insertion into the skin, the microneedle and the patch layer may be separated from each other by at least one of dissolution of the microneedle inserted into the skin and deterioration in mechanical length by swelling of the microneedle.

In one embodiment, the microneedle patch may be a patch for implanting a microneedle of implanting the microneedle to the skin.

In one embodiment of the microneedle may include a drug.

Another aspect of the present invention provides a manufacturing method for a microneedle patch including: injecting a polymer solution having at least one of swellability and solubility to a microneedle mold including a flat surface and an intaglio of the microneedle formed on the flat surface; forming a polymer layer which is covered to entirely connect the surface of the flat surface of the microneedle mold and the surface of each intaglio of the microneedle and not completely filled in each intaglio of the microneedle by solvent-casting while centrifuging the microneedle mold injected with the polymer solution; attaching a flat film on the flat surface of the microneedle mold where the polymer layer is formed; and attaching a flat film on the flat surface of the microneedle mold where the polymer layer is formed; and separating the polymer layer attached with the flat film from the microneedle mold.

### [Advantageous Effects]

According to the microneedle patch and the manufacturing method therefor of the present invention, it is possible to easily provide a microneedle patch including a microneedle including an air-pocket therein. In the microneedle patch of the present invention, when the microneedle is formed of a polymer having at least one of swellability and solubility to be applied to the skin, the microneedle is swollen in the skin to fill a tissue damage (hole) caused by inserting the microneedle. Further, after the insertion of the skin, the microneedle is dissolved to increase the viscosity, thereby increasing a stay time in the skin tissue for the material in which the microneedle is dissolved, and preventing penetration of external materials. Therefore, the microneedle patch of the present invention can prevent damage and secondary infection of the tissue, and the microneedle can carry functional materials such as drugs to slowly release the functional materials in the skin, thereby delivering drugs into the body for a long time. In addition, the microneedle may be separated from the microneedle patch to be used as an implantable drug delivery system.

### [Description of Drawings]

FIG. 1 is a diagram for explaining a microneedle patch of the present invention.
FIG. 2 is a schematic diagram for explaining the microneedle patch of the present invention.
FIG. 3 is a diagram for explaining a manufacturing method for a microneedle patch of the present invention.
FIG. 4 is a diagram for explaining a microneedle patch according to an embodiment of the present invention.

### [Modes of the Invention]

Hereinafter, embodiments of the present invention will be described with reference to the accompanying drawings. A method for manufacturing a low friction member according to an embodiment of the present invention will be described in detail with reference to the accompanying drawings. However, this does not limit the present invention to specific exemplary embodiments, and it should be understood that the present invention covers all the modifications, equivalents and replacements included within the idea and technical scope of the present invention. In describing each drawing, reference numerals refer to like elements.

Terms used in the present application are used only to describe specific exemplary embodiments, and are not intended to limit the present invention. A singular form may include a plural form if there is no clearly opposite meaning in the context. In the present application, it should be understood that term "include" or "have"indicates that a feature, a number, a step, an operation, a component, a part or the combination thereof described in the specification is present, but does not exclude a possibility of presence or addition of one or more other features, numbers, steps, operations, components, parts or combinations thereof, in advance.

If it is not contrarily defined, all terms used herein including technological or scientific terms have the same meanings as those generally understood by a person with ordinary skill in the art. Terms which are defined in a generally used dictionary should be interpreted to have the same meaning as the meaning in the context of the related art, and are not interpreted as an ideal meaning or excessively formal meanings unless clearly defined in the present application.

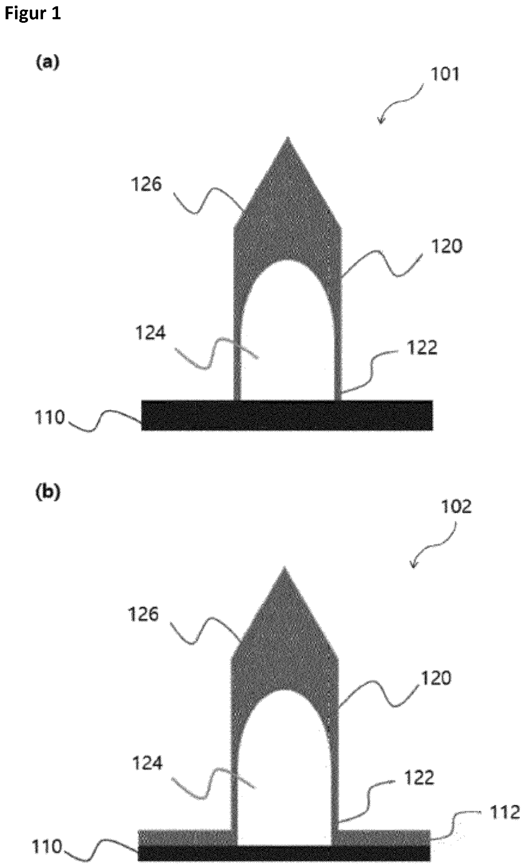

FIG. 1 is a diagram for explaining a microneedle patch of the present invention.

FIG. 1A is a diagram for explaining a microneedle patch according to an embodiment of the present invention, and FIG. 1B is a diagram for explaining a microneedle patch according to another embodiment of the present invention.

Referring to FIG. 1A, a microneedle patch 101 of the present invention includes a patch portion and a microneedle 120 disposed on one surface of the patch portion and having an air-pocket therein, and formed on a polymer having at least one of swellability and solubility.

At this time, the patch portion may be a single layered structure of a patch layer 110. The patch layer 110 may be a flat layer including a flat surface and may be a portion located on the skin surface without being inserted into the skin when the microneedle patch 101 is applied to the skin. At this time, one side of the patch layer 110 on which the microneedle 120 is formed is in contact with the skin surface. The patch layer 110 may exhibit elasticity and flexibility, and thus the shape may be flexibly deformed according to a curved skin surface. Although not illustrated, a plurality of microneedles 120 may be disposed on one surface of the patch layer 110.

The microneedle 120 refers to a needle having a fine size. A tip 126 of the microneedle refers to a portion including a point, that is, a sharp end of the microneedle 120, and a lower end 122 of the microneedle may refer to a portion which corresponds to the tip 126 of the microneedle 120 and is in contact with the patch layer 110. Although the shape of the microneedle 120 of the present invention is illustrated in FIG. 1, the present invention is not limited thereto.

The microneedle 120 includes an air-pocket 124 inside the lower end 122 of the microneedle which is in contact with the patch layer 110. The air-pocket 124 refers to a hollow hole formed in the solid and may be referred to as an air-pocket and an air bubble. The air-pocket 124 may be formed in the lower end 122 of the microneedle rather than the tip 126 of the microneedle, and accordingly, the patch layer 110 in the lower end 122 of the microneedle corresponding thereto may be exposed to the air-pocket 124.

The lower end 122 of the microneedle where the air-pocket 124 is located may have shape such as a wall in which the polymer constituting the microneedle 120 surrounds the internal air-pocket 124. At this time, the polymer wall located on the side of the air-pocket 124 may be referred to as a side wall of the microneedle 120 where the air-pocket 124 is located. In other words, the side wall may refer to a wall formed of a polymer constituting the lower end of the microneedle located on the left and right sides of the air-pocket.

The air-pocket 124 may have a shape which protrudes from the patch layer 110 to the microneedle tip 126 in the lower end 122 of the microneedle, and an exemplary shape of the air-pocket 124 is illustrated in FIG. 1, but the present invention is not limited thereto. At this time, the protruding height of the air-pocket 124 may be equal to or larger than a thickness of a skin horny layer. The horny layer of the skin is the outermost layer of the epidermis constituting the skin and serves to prevent the skin from an external environment and prevent evaporation of water in the body to maintain the homeostasis of the human body. The epidermis where the horny layer is located varies in thickness from the thinnest eyelid (mean 0.04 mm) to the thickest hand or soles (mean 1.6 mm) and usually has a thickness of 0.05 mm to 0.1 mm, and the horny layer has a thickness of an average of 0.01 to 0.03 mm (10 µm to 30 µm). Therefore, the air-pocket 124 of the microneedle 120 of the present invention may have a height protruding from the patch layer 110 of 10 µm or more, which is equal or larger than the minimum thickness of the horny layer. For example, the protruding height of the air-pocket 124 may be above the thickness of the horny layer and in the range of 10 µm to 300 µm, which can reach the dermal layer below the epidermis. Since the air-pocket 124 of the microneedle 120 of the present invention protrudes above the thickness of the horny layer, the microneedle 120 is inserted into the skin tissue when the microneedle patch 101 is attached to the skin, and then a part of the air-pocket 124 disposed therein passes through the horny layer to be located in the skin tissue below the horny layer and the remaining air pocket 124 may be disposed on the horny layer. As a result, in the microneedle 120 of the present invention, the microneedle tip 126 below the air-pocket 124 passes through the horny layer and present in the skin tissue below the horny layer. From this, the microneedle 120 and the microneedle tip 126 of the present invention may be stably and firmly fixed in the body, and the drug may be efficiently delivered into the skin tissue. In addition, in the microneedle 120 of the present invention, the side wall of the air-pocket 124 located over the horny layer is cut and/or dissolved to easily implant the microneedle tip 126 to the skin tissue below the horny layer. A detailed description thereof will be described below in more detail.

The microneedle 120 of the present invention may be formed of a polymer having at least one of swellability and solubility. The swellability refers to a property of increasing the volume by absorbing a liquid such as water. In the present invention, a 'swellable polymer' refers to a polymer having swellability as a main property, and it is not meant that the swellable polymer of the present invention does not have other properties such as solubility other than the swellability. That is, the swelling polymer of the present invention has swellability as a main property, and may exhibit other properties. For example, the swellable polymer of the present invention may exhibit both swellability and solubility. Further, in the present invention, a "soluble polymer" refers to a polymer having solubility as a main property, which is a property dissolved in a solvent. As described above, the soluble polymer of the present invention may exhibit properties other than solubility. For example, the soluble polymer may exhibit both solubility and swellability. The polymer of the present invention may be a polymer which a soluble by a body fluid, or may be a biodegradable polymer that can be decomposed by body fluids, enzymes, microorganisms, or the like in vivo. In addition, the polymer may be a biocompatible polymer having no toxicity to the human body and suitable for application to living bodies.

For example, the polymer may be at least one material selected from the group consisting of hyaluronic acid, alginic acid, pectin, carrageenan, chondroitin (sulfate), dextran (sulfate), chitosan, polylysine, collagen, gelatin, carboxymethyl chitin, fibrin, agarose, pullulan, cellulose, polyethylene oxide, poly(N-)isopropylacrylamide (PNIPAAm), polyacrylamide (PAAm), polymethacrylic acid, polymaleic acid, polyvinyl alcohol (PVA), polyethylene oxide (PEO), poly(N-)vinyl pyrrolidone (PVP), poly(methyl methacrylate-co-hydroxylethyl methacrylate) (Poly(MMA-co-HEMA)), poly(acrylonitrile-arylsulfonate), poly(glucosyloxyethyl methacrylate-sulfate) (P(GEMA)), polyethylene oxide (PEO)-polyester derivatives, a polyethylene oxide-polypropylene oxide-polyethylene oxide (PEO-PPO-PEO) ternary block copolymer, N-carboxyanhydride, polystyrene, and any copolymer of monomers forming these polymers.

The microneedle 120 of the present invention may be swollen by absorbing the body fluid in the skin tissue when inserted into the skin so that the swollen microneedle 120 may be firmly and mechanically fitted with the skin tissue. That is, when the microneedle patch 101 of the present invention is attached to the skin, the microneedle 120 is swollen in the skin tissue to be firmly mechanically fitted with the skin tissue. At this time, a shear force may be applied to the microneedle 120 inserted into the skin by a restoring force of the skin. For example, when the polymer of the present invention is a polymer having a number average molecular weight of the polymer is 10,000 or more, the microneedle 120 of the present invention is slowly dissolved in body tissue by the body fluid to form a dissolved solution (material) having a high viscosity. The dissolved solution having the high viscosity may stay in the body for a longer time than a relatively low viscosity due to the high viscosity and may restrictively prevent the penetration of materials from the outside.

After the microneedle patch 101 of the present invention is inserted to the skin, the microneedle 120 inserted to the skin may be configured to be separated from the microneedle patch 101. For example, the microneedle 120 inserted to the skin may be configured so that the side wall of the microneedle 120 where the air-pocket 124 is located is cut, by a force to be fixed to the skin by swelling and a force for removing the microneedle patch 101 from the skin. Particularly, when the microneedle patch 101 inserted to the skin is detached from the skin, the swollen micro needle 120 may be separated from the microneedle patch 101 by a force generated by an operation of removing the microneedle patch 101 from the skin, a shear force applied to the inserted microneedle 120, and a fixing force to be fixed to the skin tissue by swelling the microneedle 120 by the body fluid. That is, the microneedle 120 and the patch portion may be separated, and as a result, a part including the tip 126 of the cut microneedle 120 is present in the skin and the patch portion includes the other part of the cut microneedle 120 to be removed from the skin.

At this time, in the microneedle 120 of the present invention, the air-pocket 124 is located only in the lower end 122 of the microneedle rather than the microneedle tip 126 to more easily separate the microneedle 120 and the patch portion from each other. Specifically, due to the air-pocket 124 located in the lower end 122 of the microneedle, the thickness of the side wall of the microneedle 120 surrounding the air-pocket 124 is smaller in the polymer than the thickness of the tip 126 of the microneedle. Accordingly, when the above-described forces are applied, it is easy to cut the lower end 122 of the microneedle near the surface of the skin, so that the microneedle 120 and the patch are easily separated from each other and thus only the microneedle 120 may be located in the body. That is, only the microneedle 120 is implanted into the skin, and the patch portion may be removed from the skin. At this time, the side wall of the microneedle 120 where the air-pocket 124 is located may be formed of a polymer that can be easily cut by the action of the above-mentioned forces, and the thickness of the side wall may be a thickness to be cut by the action of these forces.

In the microneedle 120 of the present invention, as described above, the air-pocket 124 may protrude from the patch portion above the thickness of the horny layer, so that only the microneedle 120 having the thin side wall where the air-pocket 124 is located is located on the horny layer and the tip 126 of the microneedle formed of only the polymer without the air-pocket 124 may be more firmly and mechanically fitted to the skin below the horny layer to be firmly fixed to the skin tissue. On the other hand, when the air-pocket 124 protrude below the thickness of the horny layer, a part of the tip 126 of the microneedle is located in the horny layer and thus the microneedle is not sufficiently swollen enough to be fixed to the skin tissue. The swollen microneedle 120 may be removed from the skin together with the patch portion without being cut and separated by the action of the above-mentioned forces.

In addition, in the microneedle patch 101 of the present invention, since only the lower end 122 of the microneedle having the thin side wall is present in the horny layer due to the air-picket 124, the lower end 122 of the microneedle may be easily cut in the horny layer by the action of the above-mentioned forces. That is, in the microneedle 120 of the present invention in which the air-picket 124 is formed above the thickness of the horny layer, the microneedle 122 and the patch portion may be more easily separated from each other, and most of the microneedle 120 may be present in the body by cutting the lower end 122 of the microneedle. Thus, the microneedle 120 may be efficiently implanted into the skin.

In addition, the microneedle patch 101 of the present invention may by configured so that the microneedle 120 and the patch portion are separate from each other by at least one of dissolution of the microneedle 120 inserted into the skin and deterioration of mechanical strength due to swelling of the microneedle 120 after inserted into the skin. For example, when the microneedle 120 is inserted into the skin, the microneedle 120 may be dissolved by the body fluid, while the patch portion is located on the skin surface without being inserted into the skin not to be decomposed. In addition, a part of the microneedle 120 without passing through the horny layer may not be decomposed. At this time, since the lower end 122 of the microneedle in which the air-pocket 124 is located may has the thin thickness of the side wall due to the air-pocket 124 located therein, the lower end 122 of the microneedle in which the air-pocket 124 is located may be dissolved relatively earlier than the microneedle tip 126 without the air-pocket. As a result, the microneedle 120 may separated from the patch portion because the lower end 122 of the microneedle in which the air-pocket 124 is dissolved relatively fast and cut. At this time, the microneedle 120 of the present invention may be separated from the patch portion without a separate physical force by decomposing and cutting the lower end 122 of the microneedle. Unlike this, while a part of the lower end 122 of the microneedle is decomposed, the microneedle 120 may be more easily separated from the patch portion by applying the above-mentioned forces. The microneedle 120 that is cut and left in the skin tissue is continuously located in the body and the patch layer 110 may be removed from the skin, and at this time, the patch portion may include a part of the microneedle 120.

In addition, the microneedle 120 can be swollen by the body fluid when inserted into the skin. As described above, the patch portion may be located on the skin surface and may not be swollen, and a part of the microneedle 120 that does not pass through the horny layer does not absorb the body fluid not to be swollen. At this time, the swollen microneedle 120 and the non-swollen microneedle 120 may has a difference in mechanical strength. In particular, the swollen microneedle 120 becomes relatively weak in mechanical strength of the polymer due to swelling, while the non-swollen microneedle 120 exhibits a relatively high mechanical strength, so that microneedle 120 may be easily cut at the fitting portion in the skin tissue.

In other words, in the microneedle 120, by at least one of dissolution by mechanical fluid and deterioration in mechanical strength by swelling, only the microneedle 120 inserted into the skin is dissolved and a difference in mechanical strength between the swollen microneedle 120 and the polymer of the non-swollen portion occurs, so that the microneedle 120 is easily cut to separate the microneedle 120 and the patch portion from each other.

In addition, the microneedle 120 may include a drug. When the microneedle 120 includes the drug, the microneedle 120 swollen in the skin tissue may release the carried drug into the skin tissue. At this time, the drug release of the microneedle 120 may be controlled depending on the crosslinking degree or the decomposition rate of the polymer. For example, the swollen microneedle 120 may release the drug slowly in the tissue.

Referring to FIG. 1B, a microneedle patch 102 of the present invention includes a patch portion and a microneedle 120 disposed on one surface of the patch portion, having an air-pocket therein, and formed on a polymer having at least one of swellability and solubility.

At this time, the patch portion may be a laminated structure of a patch layer 110 and a needle layer 112 disposed on one surface of the patch layer 110 and a microneedle 120 may be disposed on an opposite surface of one surface of the needle layer 112 which is in contact with the patch layer 110.

Since the patch layer 110, the microneedle 120, the air-pocket 124, and the polymer are substantially the same as those described above, a detailed description thereof will be omitted, and differences will be described below.

The needle layer 112 may be a flat layer disposed on one surface of the patch layer 110 and a microneedle 120 may be disposed on a surface not in contact with the patch layer 110. When the microneedle patch 102 is attached to the skin, the surface of the needle layer 110 on which the microneedle 120 is disposed may be a surface in contact with the skin.

The air-pocket 124 of the microneedle 120 may be located in the lower end 122 of the microneedle which is in contact with the needle layer 112. Specifically, the air-pocket 124 may be formed inside the lower end 122 of the microneedle in the lower end 122 of the microneedle and on the needle layer 122 located in the lower end of the microneedle 122 corresponding thereto. That is, as illustrated in FIG. 1B, the lower end 122 of the microneedle and the needle layer 112 are in contact with each other and connected to each other, and the air-pocket 124 is formed in the lower end 122 of the microneedle and the needle layer 122 corresponding thereto so that the patch layer 110 in the lower end 122 of the microneedle may be exposed to the air-pocket 124.

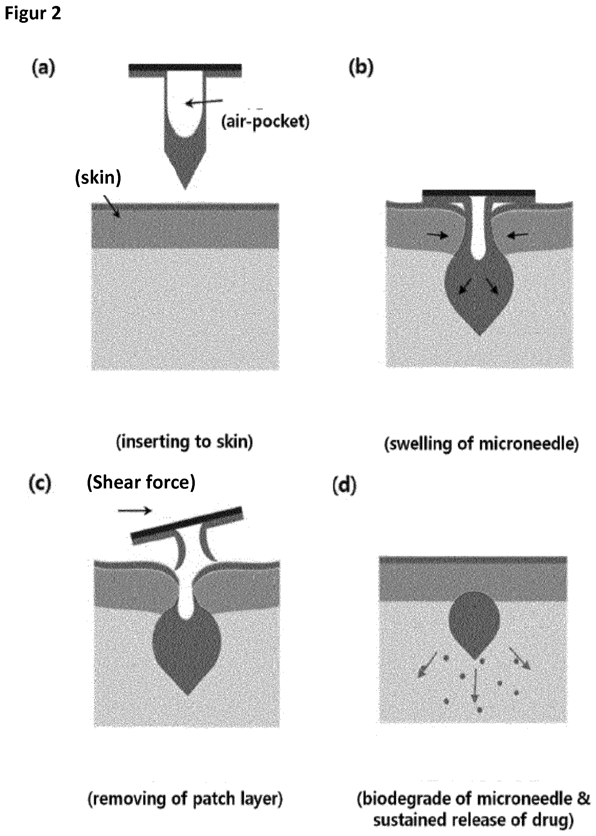

The microneedle patch 102 may be configured to separate the microneedle 120 and the patch portion substantially the same as described in the microneedle patch 101. At this time, the patch portion of the microneedle patch 102 includes both the patch layer 110 and the needle layer 112, and the needle layer 112 of the separated patch portion may include a part of the cut microneedle 120. A detailed description thereof will be described with reference to FIG. 2.

FIG. 2 is a schematic diagram for explaining the microneedle patch of the present invention.

FIG. 2A is a schematic diagram for explaining application of the microneedle patch 102 of the present invention to the skin, and FIG. 2B is a schematic diagram of swelling of the microneedle patch 102 inserted into the skin tissue. In addition, FIG. 2C is a schematic diagram for explaining removal of the patch portion of the microneedle patch 102, and FIG. 2D is a schematic diagram of swelling of the microneedle patch 102 impregnated into the skin tissue after removing the patch portion.

Although FIG. 2 illustrates a shape of the microneedle and exemplifies the microneedle patch 102 of FIG. 1B, microneedle patches 101 and 102 of the present invention will be described with reference to FIG. 2.

Referring to FIG. 2 together with FIG. 1, when the microneedle patches 101 and 102 are applied as described in the microneedle patch 101 of FIG. 1A, microneedles 120 may be inserted into the skin tissue and the patch portions may be located on the skin surface. At this time, a portion of the microneedle 120 that passes through the horny layer of the skin is swollen by absorbing the body fluid and tightly mechanically fitted with the skin tissue, while a portion of the microneedle 120 located in the horny layer 120 may not be swollen. In the microneedle 120 passing through the horny layer of the skin, the size of an air-pocket 124 is equal to or larger than the thickness of the horny layer of the skin, and thus a portion of the air-pocket 124 formed in the microneedle 120 may be present in the skin tissue below the horny layer by passing through the horny layer. At this time, the tip portion 126 of the microneedle passing through the horny layer of the skin is formed only of the polymer without the air-pocket to be swollen by absorbing the body fluid and firmly mechanically fitted with the skin tissue. However, the microneedle 120 where the air-pocket 124 is located may be relatively less swollen than the tip 126 of the microneedle due to a thin thickness of the side wall by the air-pocket 124 to have a difference in a mechanical fitting degree with the skin tissue as compared to the microneedle tip 126. In addition, the microneedle 120 inserted into the skin may be applied with a shear force due to the restoring force of the skin tissue. At this time, when an operation of removing the microneedle patches 101 and 102 applied to the skin from the skin is performed, a force of removing the microneedle patches 101 and 102 is applied to the microneedle 120 together with the shear force due to the restoring force of the skin, and at this time, the microneedle 120 inserted into the skin tissue may be firmly mechanically fitted within the skin tissue by swelling and fixed into the skin. However, since the side wall of the lower end 122 of the microneedle where the air-pocket 124 is located has a relatively thin thickness, the microneedle may be cut by the action of the forces described above.

That is, by the operation of removing the microneedle patches 101 and 102 from the skin, a part of the microneedle 120 may be separated from the microneedle patches 101 and 102, a part of the separated microneedle 120 is located in the skin tissue, and the patch portion may be removed from the skin together with the part of the cut microneedle 120.

Further, the microneedle 120 inserted into the skin is dissolved, and the patch portion not inserted into the skin may not be dissolved. At this time, the microneedle 120 inserted into the skin may be relatively rapidly dissolved because the lower end 122 of the microneedle in which the air-pocket 124 is located has a thin thickness of the side wall due to the air-pocket 124. That is, a part of the microneedle 120 where the air-pocket 124 is located may be first dissolved so that the microneedle 120 may be cut, and thus the patch part and the microneedle 120 are cut, so that the patch portion may be removed from the skin. At this time, a part of the cut microneedle 120 may be continuously dissolved while being located in the skin.

Therefore, in the microneedle patches 101 and 102 of the present invention, the microneedle 120 may be cut by dissolving and swelling of the microneedle 120 in the skin, so that the microneedle 120 and the patch portion may be separated from each other. In addition, the separated microneedle 120 is still present in the skin tissue, and the patch portion may be removed from the skin. The cut microneedle 120 may be the lower end 122 of the microneedle having the air-pocket formed therein, and the patch portion may include a part of the cut microneedle 120, so that the patch portion and a part of the cut microneedle 120 may be removed.

After removing the patch portions of the microneedle patches 101 and 102 of the present invention from the skin, the skin restores the damaged tissue by the restoring force and as a result, the microneedle 120 separated in the skin tissue is impregnated and continuously located in the skin tissue. At this time, the microneedle 120 is continuously swollen in the skin tissue to fill the damage (hole) to the skin tissue generated by the microneedle 120 by applying the microneedle patches 101 and 102 to the skin, thereby preventing secondary infection by the wound.

In addition, in the microneedle patches 101 and 102, only the swollen microneedle 120 is separated to be located3in the skin tissue, so that the microneedle patches 101 and 102 may be configured as a patch for implanting the microneedle 120 of implanting the microneedle 120 into the skin. For example, when the microneedle 120 carries a functional material such as a drug, the microneedle patches 101 and 102 may be used as an implantable drug delivery system. At this time, the microneedle 120, which is impregnated and swollen into the skin tissue, is biodegraded and may release the carried drug into the skin tissue.

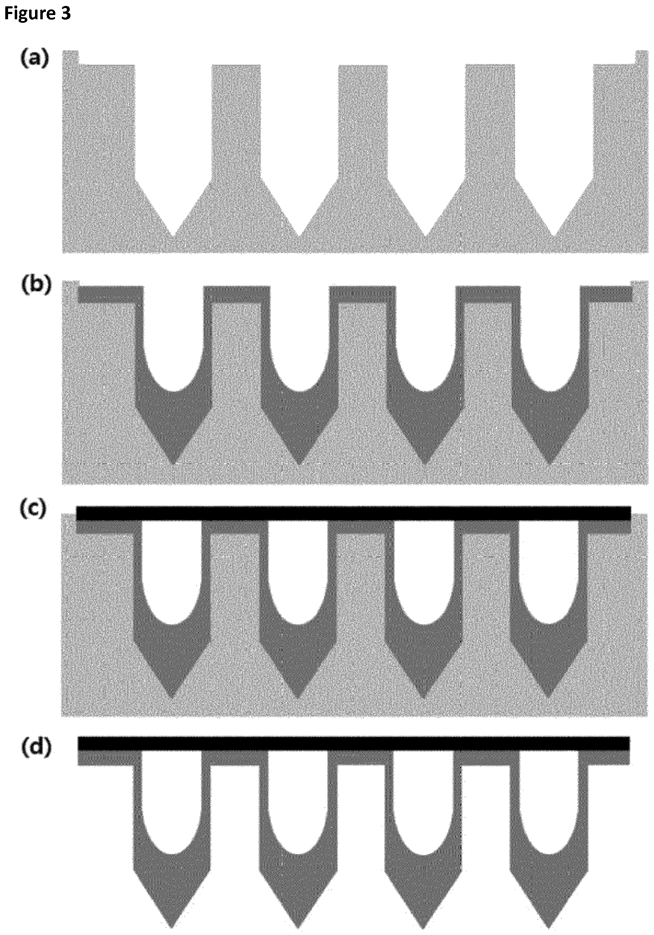

Hereinafter, a manufacturing method of a microneedle patch of the present invention will be described with reference to FIG. 3.

FIG. 3 is a diagram for explaining a manufacturing method for a microneedle patch of the present invention.

Referring to FIG. 3, in order to manufacture a microneedle patch 100 of the present invention, a polymer solution having at least one of swellability and solubility is injected into a microneedle mold (FIG. 3A).

Since the polymer is substantially the same as that described in the microneedle patches 101 and 102 of the present invention, a detailed description thereof will be omitted.

The microneedle mold has a microneedle mode includes a flat surface and an intaglio of the microneedle 120 having substantially the same shape as the microneedle 120 of the present invention formed on the flat surface. The microneedle mold is illustrated as an example in FIG. 3A, but is not limited thereto.

Then, the microneedle mold in which the polymer solution is injected is solvent-cast while being centrifuged (see FIG. 3B).

The solvent casting is a method of applying a solution and drying the solvent to form a film, and in the present invention, the microneedle mold in which the polymer solution is injected is solvent-cast while being centrifuged and covered so as to entirely connect the surface of the flat surface of the microneedle mold and the surface of each intaglio of the microneedle to form a polymer layer which is not completely filled in each intaglio of the microneedle.

Specifically, while the microneedle mold injected with the polymer solution is centrifuged, the solvent is evaporated, so that the polymer solution is concentrated outside the microneedle mold rotated by the centrifugal force by the rotating force and the centrifugal force by the rotation of the microneedle mold. Accordingly, in each of the microneedle intaglios of the microneedle mold, the microneedle tip portion of the intaglio may be completely filled with the polymer solution. On the other hand, the inside of the rotating microneedle mold may be in contact with a relatively small amount of the polymer solution. As a result, by the centrifuging and solvent-casting, a polymer layer may be formed in which each intaglio of the microneedle of the microneedle mold is not completely filled and the surface of the flat surface of the microneedle mold and the inner surface of each intaglio of the microneedle are entirely covered. At this time, the polymer layers formed on the flat surface and the inner surface of the intaglio of the microneedle may be seamlessly connected to be formed integrally. That is, as illustrated in FIG. 3B, a polymer layer, which entirely covers the surface of the microneedle mold, but has an empty space formed in each intaglio of the microneedle of the microneedle mold, may be formed. At this time, according to the present invention, conditions such as a kind of polymer, the viscosity of the polymer, the injection amount of the polymer, the centrifugation speed and the like may be arbitrarily selected by those skilled in the art so that the solvent is evaporated and the solute forms the polymer layer as described above by the centrifuging and solvent-casting.

In addition, a part of the polymer layer formed on the intaglio of the microneedle or an uncured polymer may be removed so that the intaglio of the microneedle further includes an empty space.

Next, a flat film is attached on the flat surface of the microneedle mold having the polymer layer (see FIG. 3C), and the polymer layer to which the flat film is attached is separated from a microneedle mold.

The flat film means a planar film, and may be films formed of various materials that may be attached to the polymer layer. As an example, the flat film may be a film formed of a material which may be crosslinked with the polymer layer. The flat film is attached on the flat surface of the microneedle mold to form an empty space of the intaglio of the microneedle and an air-pocket in the polymer layer of the intaglio of the microneedle by the film.

A microneedle patch of the present invention may be manufactured by separating the polymer layer attached with the flat film from the microneedle mold.

Hereinafter, a microneedle patch of the present invention will be described with reference to a specific embodiment.

A microneedle patch according to Example 1 of the present invention was manufactured using a polystyrene-polyacrylamide (PS-PAA) polymer as a polymer.

The PS-PAA polymer was injected into the microneedle mold and dried by centrifugation. Then, a polymer film was attached to manufacture a microneedle patch (hereinafter referred to as a microneedle patch) according to Example 1 of the present invention.

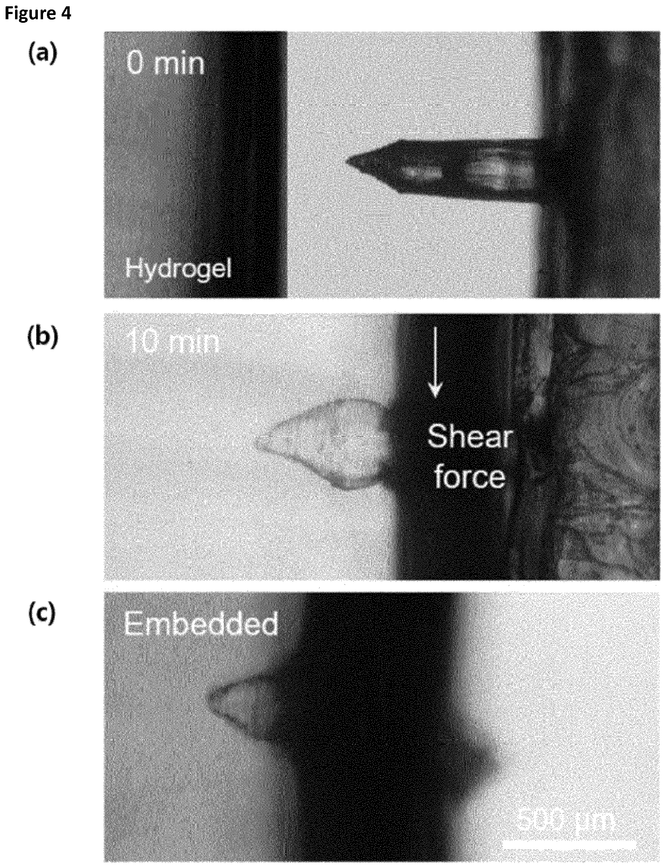

Then, the microneedle patch was inserted into the artificial skin made of a hydrogel and a change over time was checked. Thereafter, the microneedle patch was then detached from the artificial skin. The results are shown in FIG. 4.

FIG. 4 is a diagram for explaining a microneedle patch according to an embodiment of the present invention.

FIG. 4A is a photograph showing a microneedle patch before insertion into the artificial skin, and FIG. 4B is a photograph showing a microneedle patch after 10 minutes of insertion into the artificial skin. In addition, FIG. 4C is a photograph showing a microneedle inserted into the artificial skin after separating a microneedle and a patch portion of the microneedle patch.

Referring to FIG. 4, when the microneedle patch is applied to the artificial skin, it can be seen that the microneedle is inserted into the artificial skin and swollen. Further, when the patch portion of the microneedle patch is removed from the skin after insertion into the artificial skin, it can be seen that a part of the microneedle is cut so that the microneedle and the patch portion are separated from each other, and the separated swollen microneedle is present in the artificial skin.

That is, when the microneedle patch is attached to the skin, the microneedle of it can be seen that the microneedle patch is swollen by absorbing moisture and the microneedle is cut so as to easily separate the patch portion. That is, it can be seen that only the swollen microneedle may be continuously located in the skin, and the patch portion may be removed from the skin. Further, as a result, it can be seen that the microneedle patch of the present invention may be constituted by a patch for implanting the microneedle of implanting the microneedle into the skin. The present invention has been described with reference to the preferred embodiments of the present invention, but those skilled in the art will understand that the present invention can be variously modified and changed without departing from the spirit and the scope of the present invention which are defined in the appended claims.

### [Explanation of Reference Numerals and Symbols]

101, 102: Microneedle patch
110: Patch layer
112: Needle layer
120: Microneedle
122: Lower end of microneedle
124: Air-pocket
126: Tip of microneedle

## Claims

1. A microneedle patch comprising:
a patch layer which is a flat layer; and
a microneedle which is disposed on one surface of the patch layer, includes an air-pocket therein, and formed of a polymer having at least one of swellability and solubility.

2. The microneedle patch of claim 1, wherein the air-pocket is disposed in a lower end of the microneedle which is in contact with the patch layer.

3. The microneedle patch of claim 1, wherein the patch layer includes a needle layer on one surface of the patch layer, and the microneedle is disposed on an opposite surface to one surface of the needle layer which is in contact with the patch layer.

4. The microneedle patch of claim 3, wherein the air-pocket is disposed in a lower end of the microneedle which is in contact with the needle layer.

5. The microneedle patch of claim 4, wherein in the lower end of the microneedle, the air-pocket is formed in the needle layer which is in contact with the lower end of the microneedle and the patch layer is exposed to the air-pocket.

6. The microneedle patch of claim 5, wherein in the lower end of the microneedle, a height of the air-pocket protruding from the exposed patch layer is equal to or larger than a thickness of the skin horny layer.

7. The microneedle patch of claim 6, wherein the protruding height of the air-pocket is 10 µm to 300 µm.

8. The microneedle patch of claim 1, wherein after the insertion into the skin, the microneedle inserted into the skin is constituted so that the side wall of the microneedle where the air-pocket is located is cut by a force of fixing the microneedle to the skin by swelling and a force of removing the microneedle patch from the skin.

9. The microneedle patch of claim 1, wherein the polymer is at least one material selected from the group consisting of hyaluronic acid, alginic acid, pectin, carrageenan, chondroitin (sulfate), dextran (sulfate), chitosan, polylysine, collagen, gelatin, carboxymethyl chitin, fibrin, agarose, pullulan, cellulose, polyethylene oxide, poly(N-)isopropylacrylamide (PNIPAAm), polyacrylamide (PAAm), polymethacrylic acid, polymaleic acid, polyvinyl alcohol (PVA), polyethylene oxide (PEO), poly(N-)vinyl pyrrolidone (PVP), poly(methyl methacrylate-co-hydroxylethyl methacrylate) (Poly(MMA-co-HEMA)), poly(acrylonitrile-arylsulfonate), poly(glucosyloxyethyl methacrylate-sulfate) (P(GEMA)), polyethylene oxide (PEO)-polyester derivatives, a polyethylene oxide-polypropylene oxide-polyethylene oxide (PEO-PPO-PEO) ternary block copolymer, N-carboxyanhydride, polystyrene, and any copolymer of monomers forming these polymers.

10. The microneedle patch of claim 1, wherein the number average molecular weight of the polymer is 10,000 or more.

11. The microneedle patch of claim 1, wherein after the insertion into the skin, the microneedle and the patch layer are separated from each other by at least one of dissolution of the microneedle inserted into the skin and deterioration in mechanical length by swelling of the microneedle.

12. The microneedle patch of claim 1, wherein the microneedle patch is a patch for implanting a microneedle of implanting the microneedle to the skin.

13. The microneedle patch of claim 1, wherein the microneedle includes a drug.

14. A manufacturing method for a microneedle patch comprising:
injecting a polymer solution having at least one of swellability and solubility to a microneedle mold including a flat surface and an intaglio of the microneedle formed on the flat surface;
forming a polymer layer which is covered to entirely connect the surface of the flat surface of the microneedle mold and the surface of each intaglio of the microneedle and not completely filled in each intaglio of the microneedle by solvent-casting while centrifuging the microneedle mold injected with the polymer solution;
attaching a flat film on the flat surface of the microneedle mold where the polymer layer is formed; and
separating the polymer layer attached with the flat film from the microneedle mold.
